(19) **Europäisches Patentamt European Patent Office Office européen des brevets**

(11) **EP 1 610 878 B1**

(12) **EUROPEAN PATENT SPECIFICATION**

(45) Date of publication and mention of the grant of the patent:
**03.09.2014 Bulletin 2014/36**

(51) Int Cl.:
*C30B 7/00* *(2006.01)*   *A61K 9/16* *(2006.01)*
*B01D 9/00* *(2006.01)*   *A61K 9/00* *(2006.01)*

(21) Application number: **04712610.7**

(22) Date of filing: **19.02.2004**

(86) International application number:
**PCT/GB2004/000654**

(87) International publication number:
**WO 2004/073827 (02.09.2004 Gazette 2004/36)**

(54) **PROCESS FOR THE PRODUCTION OF PARTICLES**

VERFAHREN ZUR HERSTELLUNG VON TEILCHEN

PROCEDE DE PRODUCTION DE PARTICULES

(84) Designated Contracting States:
**AT BE BG CH CY CZ DE DK EE ES FI FR GB GR HU IE IT LI LU MC NL PT RO SE SI SK TR**

(30) Priority: **21.02.2003 GB 0304037**
**06.06.2003 GB 0313129**

(43) Date of publication of application:
**04.01.2006 Bulletin 2006/01**

(73) Proprietor: **UNIVERSITY OF BATH**
**Claverton Down,**
**Bath, BA2 7AY (GB)**

(72) Inventors:
• **PRICE, Robert**
**Llanvair Discoed,**
**Monmouthshire NP16 6LW (GB)**
• **KAERGER, Joerg, Sebastian**
**D-04420 Markranstaedt (DE)**

(74) Representative: **Mansfield, Peter Turquand**
**Coller IP Management Limited**
**Fugro House**
**Hithercroft Road**
**Wallingford, Oxfordshire OX10 9RB (GB)**

(56) References cited:
**WO-A-01/66090      WO-A-01/87277**
**WO-A-02/089942    US-B1- 6 482 438**

**Description**

This invention relates to a process for the production of particles and to the particles themselves. The invention has particular application in the field of pharmaceutical formulations, especially inhalation formulations, and in the field of agrochemical formulations, especially liquid-based suspensions.

A number of drugs are available to treat patients with asthma or other respiratory disorders. One preferred way of administering such drugs is by inhalation, and a number of different types of inhaler, specifically metered dose inhalers, nebulisers, and dry powder inhalers, are available.

The size and surface properties of the particles used in a pharmaceutical formulation can play a crucial role in the behaviour of the particles. The therapeutic efficacy of suspension based pressurised metered dose inhalers and dry powder inhalation formulations depends critically upon the physical properties of the particles being inhaled. In general, drug particles of relatively small size are carried by carrier particles of a much larger particle size. On inhalation, the smaller drug particles are stripped off from the carrier particles and carried into the lungs. The particle size range of the solid active ingredient particularly affects the efficacy of the formulation. For example, optimum particle size for delivery of $\beta$2-agonist is 6 $\mu$m and for peptides it is 0.5 to 2$\mu$m. Particle size can also affect the therapeutic efficacy of pharmaceutical solid dosage forms as particle size alters the solubility profiles of low solubility drug particles for oral administration. It is, therefore, desirable to be able to produce drug particles for use in pharmaceutical formulation with a narrow particle size distribution about the optimum particle size for therapeutic efficacy.

WO02/089942 describes a method for the formation of crystalline particles, where a solution of a desired substance is mixed with an anti-solvent, and the resultant mixture is subjected to high intensity ultrasound to induce crystal formation.

Generally, drug particles for inhalers are produced by mechanical micronisation whereby opposing jets of compressed air force particles to impact with one another generating particles of reduced size. Particles produced by mechanical micronisation are adversely affected by such a high energy process and the resultant particles exhibit increased surface rugosity, surface free energy, electric charge and, most significantly, degree of crystalline disorder. The disordered regions are thought to be primarily on the particle surfaces and are metastable. Under certain conditions, in particular when subjected to conditions of elevated temperature or humidity, the amorphous surface regions on particles may undergo a recrystallisation process, possibly causing particle agglomeration. Thus, the stability and performance of the drug particles during storage and handling are influenced by the effects of the mechanical micronization process. Furthermore, mechanical micronization does not facilitate control of the surface properties of particles. There is, therefore, a need for a process that enables greater control of particle size and particle size distribution and produces particles that are stable during storage and handling.

Alternative techniques for the production of drug particles include the generation of an aerosol of droplets from a solution of the drug and subsequent spray drying of the droplets to solidify the particles. The disadvantage of the spray drying technique is that the particles tend to be 100% amorphous rather than crystalline as the solidification is rapid. Freeze drying of aerosol droplets has also been used to obtain particles but, again, the rapid solidification leads to the generation of amorphous particles.

Agrochemical formulations are commonly applied by spraying, usually as a water based spray formulation. One formulation type is a suspension of solid particles including an agrochemical active (usually a water insoluble active) in a liquid medium, commonly initially formulated as a concentrate (a suspension concentrate) which is diluted before use as a spray.

The present invention provides a process for the production of particles comprising the steps of

   i) formation of a solution of a desired substance in a suitable solvent;
   ii) generation of an aerosol from the solution of said substance;
   iii) collection of the aerosol droplets in a vessel containing a non-solvent of said substance; and iv) application of ultrasound to the droplets dispersed in the non-solvent to effect crystallisation of said substance.

The process of the invention may be used to prepare particles of any desired soluble substance. It may for example be used to prepare particles suitable for use in a pharmaceutical formulation, for example any soluble pharmaceutically acceptable substance including both pharmaceutically active ingredients and pharmaceutical excipients and is especially applicable to the production of drug particles. In a further preferred embodiment, the process according to the invention may be used to prepare particles suitable for use in an agrochemical formulation; it may be used to prepare particles of any soluble agrochemically active substance, especially for use in the agrochemical formulation types mentioned above. Therefore, preferably, the substance of which particles are prepared using the process of the invention is a pharmaceutically acceptable substance or an agrochemically active substance, although other types of substance may also be used.

Any aerosol based atomisation system may be used for generation of the aerosol. Various systems for generating aerosols are well-known. The aerosol may, for example, be generated from the desired substance dissolved in a suitable solvent by electrohydrodynamic spraying, high air pressure atomiser or other aerosol generators including pneumatic systems, rotary (spinning-top) systems, spray nozzles, nebulizers, propellant evaporation systems, piezoelectric transducers and ultrasonic transducers.

In the present invention, controlling the properties of the aerosol allows control of the nature of the particles generated. The aerosol is generally a micrometer sized aerosol and the production of the aerosol is controlled such that the droplets of the aerosol are of the appropriate size range to produce particles appropriate for the desired purpose. However, the process may also be used to prepare nano-sized particles, in which case nano-sized aerosol droplets may be generated.

The size and dispersity of the droplets of the aerosol critically affect the size and dispersity of the particles, e.g. drug particles, produced by the process of the invention. The size of the droplets is preferably from 10nm to 1mm, especially from $1\mu m$ to $200\mu m$, and most preferably from $1\mu m$ to $50\mu m$ and may, for example, be controlled by varying the diameter of the nozzle through which the droplets are ejected. If nanometre sized particles are desired, aerosol droplets of 10nm to $1\mu m$ will be preferred. The degree of polydispersity of the droplets is preferably within the required dispersity of the desired crystalline particles. Appropriate dispersity measurements can be made by standard techniques such as laser light scattering measurements.

Between the ejection of the droplets from the aerosol generator and the time the droplets reach the surface of the non-solvent, solvent evaporates from the droplets. Thus the composition of the droplets changes between formation of the droplets and collection of the droplets in the non-solvent. Evaporation of the solvent increases the concentration of the desired substance in the droplet and the droplet may be a super-cooled melt of the substance by the time it reaches the surface of the non-solvent. Generally, a proportion of the droplets may be 100% of the desired substance, for example 100% drug molecules, by the time they reach the non-solvent whilst the remainder of the droplets will contain both molecules of the desired substance and solvent molecules. Droplets with a high concentration of the desired substance yield particles which are more spherical than particles obtained as crystals via crystal growth from supersaturated solutions. Preferably the process parameters are controlled such that on reaching the surface of the non-solvent at least 80% by mass of each droplet is the desired substance, more preferably at least 90% by mass of each droplet is molecules of the desired substance and most preferably at least 95% by mass of each droplet is molecules of the desired substance.

Varying the concentration of the desired substance in the solution prepared in step (i) of the process of the invention affects the resultant particle size. As increasing the concentration increases the number of molecules of the desired substance in a droplet, the particle size of the resultant particles increases with concentration; thus concentration can be selected to adjust the size of the particles produced. Preferably the concentration of the desired substance in the solution used to generate the aerosol is at least 0.1mg/ml, more preferably the concentration is 10mg/ml to 500mg/ml and most preferably the concentration is 50mg/ml to 200mg/ml.

Increasing the concentration decreases the amount of solvent in the droplets and increases the proportion of droplets that have completely evaporated their solvent upon collection.

The extent of solvent evaporation also depends upon the temperature of the solution, the temperature of the nozzle and the temperature during evaporation, the separation distance between the point of ejection of the droplets and the surface of the non-solvent and the type of solvent used. Increasing the temperature increases the extent of solvent evaporation. The greater the separation between the point of ejection of the droplet and the surface of the non-solvent, the greater the evaporation of the solvent. Thus, the extent of evaporation of the solvent can also be varied by varying the separation distance.

The temperature, solvent and separation distance may, therefore, be selected with regard to each other to achieve the appropriate conditions for suitable evaporation.

Obviously, the actual solvent used will also determine how rapidly evaporation of the solvent occurs. Suitable solvents depend on the solubility of the substance of interest in any particular solvent and include those listed in Table 1.

Table 1

| Solvent | Vapour pressure (units Pa or Nm$^{-2}$) |
|---|---|
| Ethanol | 5.8 kPa |
| Methanol | 12.3 kPa |
| Acetone | 24 kPa |
| Cyclohexane | 12.7 kPa |
| Acetonitrile | 9.6 kPa |
| Diethylether | 58.6 kPa |

(continued)

| Solvent | Vapour pressure (units Pa or Nm$^{-2}$) |
|---|---|
| Acetaldehyde | 99 kPa |
| Diethylester | 10 kPa |

Other suitable solvents may include: industrial methylated spirits, isopropanol, dimethylsulfoxide and highly volatile liquefied propellants including n-butane, propane, isobutane, dimethylether, chlorofluorocarbon 12, hydrofluorocarbon 134a, hydrofluorocarbon 227, HCFC-22, HCFC-123, HCFC-124 and hydrofluoroalkanes such as HFA-125 (pentafluoroethane) and HFA-152 (difluoroethane). High volatility solvents will be more likely to evaporate completely and generate a larger proportion of droplets that are 100% desired substance on reaching the non-solvent surface. Mixtures of any of the solvents may be used. For example, mixtures may be used to increase the solubility of the desired substance, e.g. drug, in the solvent or adjust the vapour pressure of the solvent and thus the extent of solvent evaporation from the droplets. It may also be possible to use supercritical solvents such as supercritical $CO_2$.

The various factors that affect solvent evaporation will also affect the viscosity of the droplets and manipulation of temperature, separation distance and solvent used allows a degree of control of the viscous properties of the droplets. The viscosity of the aerosol droplets requires careful control because the viscosity must be high enough that when the droplet impacts on the non-solvent surface, the droplet does not spatter or break up into smaller droplets or deform from its overall spherical form. If insufficient solvent evaporation occurs prior to collection of the droplets in the non-solvent, the droplets may be flattened in comparison with the more spherical droplets in which complete evaporation of the solvent has occurred by the time the droplets are collected in the non-solvent. Conversely, however, the viscosity needs to be low enough to allow the molecules of the desired substance to freely diffuse in the droplet so that on being subjected to ultrasonic energy the molecules are able to overcome the rate limiting kinetic nucleation barrier. If the viscosity of the droplet is above the viscosity associated with the glass transition temperature of the desired substance, particles with an amorphous content will result. Mixtures of solvents may be used to obtain a suitable viscosity in the collected droplets.

The aerosol droplets are collected in a non-solvent of the desired substance. Preferably the solubility of the desired substance in the non-solvent is not more than 0.1% w/w. The non-solvent is preferably a low volatility non-solvent. Preferably, if all of the solvent has not evaporated from the aerosol droplets upon collection in the non-solvent, the solvent is miscible with the non-solvent. Examples of suitable miscible solvents and non-solvent pairs are listed in Table 2.

Table 2

| Preferred Solvent (also possible non-solvent) | Preferred Non-solvent (also possible Solvent) |
|---|---|
| Methanol | Water |
| Acetone | Water |
| Ethanol | Water |
| Ethanol | Cyclohexane |
| Methanol | Cyclohexane |
| Ethanol | Isopropanol |
| Methanol | Isopropanol |
| Acetone | Isooctane |
| Dimethylsulfoxide | Water |
| Industrially methylated spirits | Water |
| Dimethylacetamide | Water |

Any of the listed solvents may also be suitable for use as a non-solvent for a particular substance and any of the listed non-solvents may also be a suitable solvent. The non-solvent may be a mixture of solvents.

An emulsifier may be added to the non-solvent to aid in the dispersion of the aerosol droplets in the non-solvent. Preferably not more than 1.0% of emulsifier is added to the non-solvent, more preferably not more than 0.5% of emulsifier is added to the non-solvent and most preferably not more than 0.1% of emulsifier is used. Suitable emulsifiers may, for example, include those listed in Table 3.

Table 3

| Emulsifier | Hydrophile- Lipophile Balance | Type of Emulsion |
| --- | --- | --- |
| Cholesterol | HLB 3 | (w/o) |
| Sodium dodecylsulfate | HLB 40 | (o/w) |
| Sorbitanmonooleate | HLB 4.3 | (w/o) |
| Tween | HLB 15-17 | (o/w) |
| Span | HLB 2-8 | (w/o) |

w/o denotes the formation of a water in oil emulsion and o/w denotes the formation of an oil in water emulsion.

The collection vessel may be a temperature-controlled collection vessel. The temperature may be varied to effect some control of the extent of molecular diffusion in the collected aerosol droplets.

Although the evaporation of the solvent from the aerosol droplets can produce supersaturated droplets of molecules of the desired substance, the substance will not crystallise in the absence of ultrasonic energy. Normally, in the prior art, freeze drying or spray drying are used to obtain particles. As stated previously, those methods generate amorphous rather than crystalline particles. In the process of the invention, ultrasonic energy is applied to the droplets upon collection of the droplets in a suitable non-solvent and induces nucleation and subsequent crystallisation of the droplets thus generating crystalline particles of the desired size.

The ultrasonic energy may be applied continuously or in a discontinuous manner such as by pulsed application. Any suitable source of ultrasonic vibration may be used. An ultrasonic probe may, for example, be inserted into the collection vessel, an ultrasonic emitter may be contained in the collection vessel or the collection vessel may be housed in an ultrasonic bath.

The amplitude & frequency of the ultrasound waves affects the rate of nucleation and crystal growth. The frequency of the ultrasound waves may for example be from 20kHz to 5MHz.

The process of the invention can be used to make particles wherein each particle comprises more than one substance. For example, particles comprising two or more drugs can be made by generating an aerosol from a solution of two or more drugs. It would then be necessary to tailor the other aspects of the process, such as solvent used, non-solvent used and temperature, with regard to both drugs.

The particles produced by the process of the invention are crystalline and have an overall shape that is substantially spherical in comparison with the morphology of particles produced by mechanical micronisation. The particles do not, however, have a perfectly smooth surface. The surface of the essentially spherical particles has nanometre scale corrugations. The shape and surface morphology of the particles produced by the process of the invention is such that the contact area between two particle surfaces differs from the contact area of prior art particles. The spherical shape and corrugated-texture of the particles is beneficial in reducing particle adhesion. The particles produced by the process of the invention lack the amorphous nature of particles produced by spray or freeze drying processes and the amorphous regions commonly found on the surface of particles produced by micronisation and, therefore, exhibit reduced inter-particle interactions so that they are not as susceptible to agglomeration. Thus the particles have greater stability during storage and handling than particles produced by the prior art methods. The particles produced by the process of the invention are characteristically more monodispersed than prior art particles in terms of their degree of skewness towards larger particle sizes and an increased peakness (leptokurtic) of the particle size distribution.

The invention further provides particles suitable for use in a pharmaceutical formulation, where the particles are crystalline and are spherical and have nanometre scale surface corrugations. The particles of the invention are drug particles suitable for use in inhalation therapy.

Typical drugs which can be formed into crystalline particles by the process of the present invention include for example corticosteroids, $\beta_2$-agonists, anticholinergics, leukotriene antagonists, inhalable proteins or peptides, mometasone furoate; beclomethasone dipropionate; budesonide; fluticasone; dexamethasone; flunisolide; triamcinolone; salbutamol; albuterol; terbutaline; salmeterol; bitolterol; ipratropium bromide; oxitropium bromide; sodium cromoglycate; nedocromil sodium; zafirlukast; pranlukast; formoterol; eformoterol; bambuterol; fenoterol; clenbuterol; procaterol; broxaterol; (22R) -6$\alpha$, 9 $\alpha$ -difluoro-11$\beta$, 21-dihydroxy-16$\alpha$, 17$\alpha$-propylmethylenedioxy-4-pregnen-3,20-dione; TA-2005; tipredane; insulin; interferons; calcitonins; parathyroid hormones; and granulocyte colony-stimulating factor.

Other medicaments which may be suitable for preparation of particles according to the invention include any drugs usefully delivered by inhalation for example, analgesics, e.g. codeine, dihydromorphine, ergotamine, fentanyl or morphine; anginal preparations, e.g. diltiazem; antiallergics, e.g. cromoglycate, ketotifen or nedocromil; anti-infectives, e.g. cephalosporins, penicillins, streptomycin, sulphonamides, tetracyclines or pentamidine; antihistamines, e.g. methapyrilene; anti-inflammatories, e.g. beclomethasone, flunisolide, budesonide, tipredane, triamcinolone acetonideor fluticasone; antitussives, e.g. noscapine; bronchodilators, e.g. ephedrine, adrenaline, fenoterol, formoterol, isoprenaline, metaproterenol, phenylephrine, phenylpropanolamime, pirbuterol, reproterol, rimiterol, salbutamol, salmeterol, terbutalin; isoetharine, tulobuterol, orciprenaline or (-)-4-amino-3,5-dichloro-α-[[[6-[2-(2-pyridinyl)ethoxy]hexyl]amino]methyl]-benzenemethanol; diuretics, e.g. amiloride; anticholinergics e.g. ipratropium, atropine or oxitropium; hormones, e.g. cortisone, hydrocortisone or prednisolone; xanthines e.g. aminophylline, choline theophyllinate, lysine theophyllinate or theophylline; and therapeutic proteins and peptides, e.g. insulin or glucagon. It will be clear to a person skilled in the art that, where appropriate, the medicaments may be used in the form of salts (e.g. as alkali metal or amine salts or as acid addition salts) or as esters (e.g. lower alkyl esters) or as solvates (e.g. hydrates) to optimise the activity and/or stability of the medicament.

Particularly preferred medicaments for preparation of particles in accordance with the invention include antiallergics, bronchodilators and anti-inflammatory steroids of use in the treatment of respiratory disorders such as asthma by inhalation therapy, for example cromoglycate (e.g. as the sodium salt), salbutamol (e.g. as the free base or as the sulphate salt), salmeterol (e.g. as the xinafoate salt), terbutaline (e.g. as the sulphate salt), reproterol (e.g. as the hydrochloride salt), beclomethasone dipropionate (e.g. as the monohydrate), fluticasone propionate or (-)-4-amino-3,5-dichloro-.alpha.-[[[6-[2-(2-pyridinyl)ethoxy]hexyl]amino]-methyl]benzenemethanol. Salmeterol, salbutamol, fluticasone propionate, beclomethasone dipropionate and physiologically acceptable salts and solvates thereof are especially preferred.

It will be appreciated that the particles of the invention may contain a combination of two or more active ingredients. Medicaments may be selected from suitable combinations of the medicaments mentioned herein before. Thus, suitable combinations of bronchodilatory agents include ephedrine and theophylline, fenoterol and ipratropium, and isoetharine and phenylephrine formulations.

Further suitable combinations of drugs include combinations of corticosteroids, such as budesonide, beclomethasone dipropionate and fluticasone propionate, with β$_2$-agonists, such as salbutamol, terbutaline, salmeterol and formoterol and physiologically acceptable derivatives thereof especially salts including sulphates. The active substance may also be a combination of the above with ipratropium bromide, especially salbutamol sulphate.

Other suitable drug compositions for use in the invention may contain bronchodilators such as salbutamol (e.g. as the free base or as the sulphate salt), salmeterol (e.g. as the xinafoate salt) or isoprenaline in combination with an antiinflammatory steroid such as a beclomethasone ester (e.g. the dipropionate) or a fluticasone ester (e.g. the propionate) or a bronchodilator in combination with an antiallergic such as cromoglycate (e.g. the sodium salt). Combinations of isoprenaline and sodium cromoglycate, salmeterol and fluticaseone propionate, or salbutamol and beclomethasone dipropionate as especially preferred.

The active substance may also be a cromone which may be sodium cromoglycate or nedocromil.

The active substance may also be a carbohydrate, for example, heparin.

The active substance may be a drug suitable for inhalation and may be a pharmacologically active agent for systemic use. For example, the active particle may comprise peptides or polypeptides or proteins such as Dnase, leukotines or insulin (including pro-insulins), cyclosporin, interleukins, cytokines, anticytokines and cytokine receptors, vaccines, growth hormone, leuprolide and related analogues, intereferons, desmopressin, immmunoglobulins, erythropoeitin and calcitonin.

Alternatively, the active substance may be suitable for oral administration. A drug for oral administration may be one of the systemic drugs mentioned above. The active substance may be a substance which exhibits low solubility in the digestive tract, for example, magnesium trisilicate, calcium carbonate and bismuth subnitrate. Organic compounds may include, for example, all products of combinatorial chemistry, rosiglitazone and other related glitazone drugs, hydrochlorothiazide, griseofulvin, lamivudine and other nuclease reverse transciptase inhibitors, simvastatin and other statin drugs, benzafibrate and other fibrate drugs and loratidine, and any other physiologically tolerable salts and derivatives thereof.

Pharmaceutical excipients suitable for preparation of particles according to the invention include, for example, carbohydrates especially monosaccharides such as fructose, glucose and galactose; non-reducing disaccharides such as sucrose, lactose and trehalose; non-reducing oligosaccharides such as raffinose and melezitose; non reducing starch derived polysaccharides products such as maltodextrins, dextrans and cyclodextrins; and non-reducing alditols such as mannitol and xylitol.

Where the particles prepared by the process of the present invention are agrochemically active, the active may for example be a plant growth regulator, herbicide, and/or pesticide, for example insecticide, fungicide, acaricide, nematocide, miticide, rodenticide, bactericide, molluscicide or bird repellant. Usually, the active will be a water insoluble or immiscible material

Examples of organic water-insoluble agrochemical active ingredients include insecticides, for example selected from the group consisting of carbamates, such as methomyl, carbaryl, carbofuran, or aldicarb; organo thiophosphates such as EPN, isofenphos, isoxathion, chlorpyrifos, or chlormephos; organo phosphates such as terbufos, monocrotophos, or terachlorvinphos; perchlorinated organics such as methoxychlor; synthetic pyrethroids such as fenvalerate; nematicide carbamates, such as oxamyl herbicides, for example selected from the group consisting of triazines such as metribuzin, hexaxinone, or atrazine; sulfonylureas such as 2-chloro-N-[(4-methoxy-6-methyl-1,3,5-triazin-2-yl)aminocarbonyl]-benzenesulfonamide; uracils (pyrimidines) such as lenacil, bromacil, or terbacil; ureas such as linuron, diuron, siduron, or neburon; acetanilides such as alachlor, or metolachlor; thiocarbamates such as benthiocarb (SATURN), triallate; oxadiazol-ones such as oxadiazon; phenoxyacetic acids such as 2,4-D; diphenyl ethers such as fluazifop-butyl, acifluorfen, bifenox, or oxyfluorfen; dinitro anilines such as trifluralin; glycine phosphonates such as glyphosate salts and esters; dihalobenzonitriles such as bromoxynil, or ioxynil; fungicides, for example selected from the group consisting of nitrilo oximes such as cymoxanil (curzate); imidazoles such as benomyl, carbendazim, or thiophanate-methyl; triazoles such as triadimefon; sulfenamides such as captan; dithiocarbamates such as maneb, mancozeb, or thiram; chloronated aromatics such as chloroneb; dichloro anilines such as iprodione; aphicides, for example selected in the group consisting of carbamates, such as pirimicarb; miticides, for example selected from the group consisting of propynyl sulfites such as propargite; triazapentadienes such as amitraz; chlorinated aromatics such as chlorobenzilate, or tetradifan; and dinitrophenols such as binapacryl.

The organic water-insoluble agrochemical active ingredients may be comprised in the particles as a mixture of several ingredients. Especially preferred organic water-insoluble agrochemical active ingredients are atrazine, cymoxanil, chlorothalanil, cyproconazole, and tebuconazole.

By way of example, certain embodiments of the invention will now be described with reference to the accompanying drawings, in which:

Fig. 1 is a schematic representation of an electrohydrodynamic spraying system
Fig. 2 is a schematic representation of a high pressure atomisation system
Fig. 3 shows the particle size distribution of paracetamol particles before and after electrohydrodynamic spraying and sonocrystallisation
Fig. 4 shows paracetamol particles before being subjected to the process of the invention
Fig. 5 shows paracetamol particles after being subjected to the process of the invention
Fig. 6 shows scanning electron micrographs of paracetamol particles produced by the air pressure atomisation embodiment of the invention with increasing drug concentration (A-B 1% w/w, C-D 5% w/w and E-F 10% w/w respectively).
Fig. 7 shows a scanning electron micrograph of BDP particles produced from a 2% w/w BDP-ethanol solution by the high pressure atomisation embodiment of the invention.
Fig. 8 shows a scanning electron micrograph of particles of crystalline budesonide produced from an 8% w/w budesonide solution in dichloromethane by the high pressure atomisation embodiment of the invention.

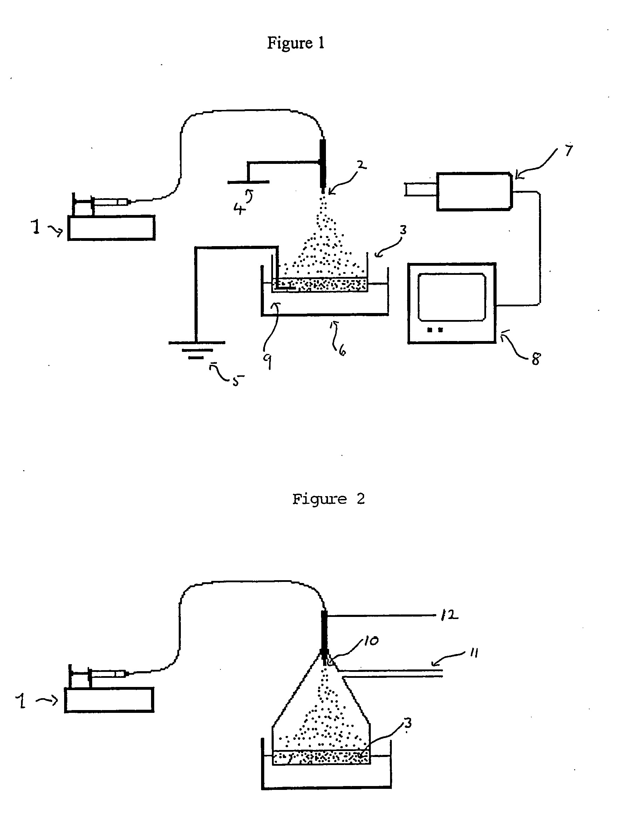

In the embodiment of the invention shown in Fig. 1, an electrohydrodynamic spraying system is used to generate the aerosol. The electrohydrodynamic spraying dispersion system comprises a single stainless steel capillary (2) or an array of stainless steel capillaries positioned vertically above a collection vessel (3) containing a non-solvent of the substance e.g. drug and a solution of the substance is forced through each capillary via a syringe pump (1) at a constant volume flow rate. Each capillary is connected to a variable high voltage supply (4). A camera (7) and monitor (8) are arranged as shown.

In order to produce the requisite monodispersed aerosol, it is necessary to generate a Taylor cone-jet by careful selection of the volume flow rate and the bias applied to the capillary needle. The size of the monodispersed droplets can be adjusted by varying the solute concentration and inner diameter of the capillary. The separation distance between the point where the droplets are ejected from the capillary and the surface of the non-solvent can also be adjusted to adjust the viscosity of the droplets. Table 4 gives suitable ranges of the variable parameters.

The highly charged aerosolised droplets are subsequently collected in the non-solvent. The non-solvent is grounded by an earthed metal electrode (5) which ensures stability of the Taylor cone and subsequent discharging of the droplets. A low concentration of an emulsifier is added to aid the dispersion of the charged droplets. The crystallisation of the supersaturated droplets is effected by the application of ultrasound via an ultrasonic emitter (6) located at the bottom of the reactor in ultrasonic bath (9).

Table 4

| Parameter | Range |
|---|---|
| Flow rate | 3$\mu$l/min to 300 $\mu$l/min |
| Applied High Voltage | 3.8kV to 15kV |
| Inner Diameter of Capillaries | 200$\mu$m to 2mm |

(continued)

| Parameter | Range |
|---|---|
| Drug Concentration | 0.1 % to Maximum solubility |
| Capillary-Solution Separation Distance | 2 to 10cm |
| Emulsifier | <0.1% |

In a further embodiment of the invention illustrated in Fig. 2, a high pressure atomisation system is used to generate an aerosol. A solution of the substance e.g. drug in an appropriate solvent is sprayed through an orifice (10) with an appropriate supporting air flow rate provided by high pressure supply 12. The flow rate of the solvent is controlled by a syringe pump (1). As in the electrohydrodynamic spraying method, the aerosol droplets are collected in a continuous phase non-solvent of the substance (3). A typical separation distance between the point the aerosol droplets are ejected and the surface of the non-solvent is around 15cm. The whole system is generally hermetically sealed with a side arm (11) attached to the flask providing a flow path of the air through the system. Nucleation of the droplets collected in the crystallisation vessel is induced via ultrasonic energy.

## Example 1 - Preparation of Crystalline Paracetamol Particles Method

The electrohydrodynamic spraying embodiment of the invention described above was used to produce paracetamol particles. A 7.5%w/w solution of paracetamol in ethanol was used to fill a syringe that was connected via tubing to capillary needles. The syringe was driven under controlled flow rates by a suitable syringe driver (Harvard PHD2000). The high voltage supply was increased until the spray from the capillary was in the form of a stable cone jet. The aerosol droplets were collected in a continuous phase in a crystallisation vessel. The continuous phase was cyclohexane with the addition of a low concentration of emulsifier. The emulsifier was added to stabilise the quasi-emulsion of the dispersed phase. Nucleation of the aerosol droplets was induced by ultrasonic energy supplied to the crystallisation vessel. The crystallised particles were collected by filtration and washed with a non-solvent via a $0.22\mu m$ filter and subsequently dried at 40°C. The experimental conditions used for generating the paracetamol particles are summarised in Table 5.

Table 5

| Parameter | Experimental Conditions |
|---|---|
| Flow rate | 70 $\mu l/min$ |
| Applied High Voltage | 9.5kV |
| Inner Diameter of Capillaries | $200 1\mu m$ - 1.6mm |
| Drug Concentration | 7.5% w/w |
| Capillary-Solution Separation Distance | 5cm |
| Emulsifier | 0.01% Span 80 |
| Non-Solvent | Cyclohexane |

## Results

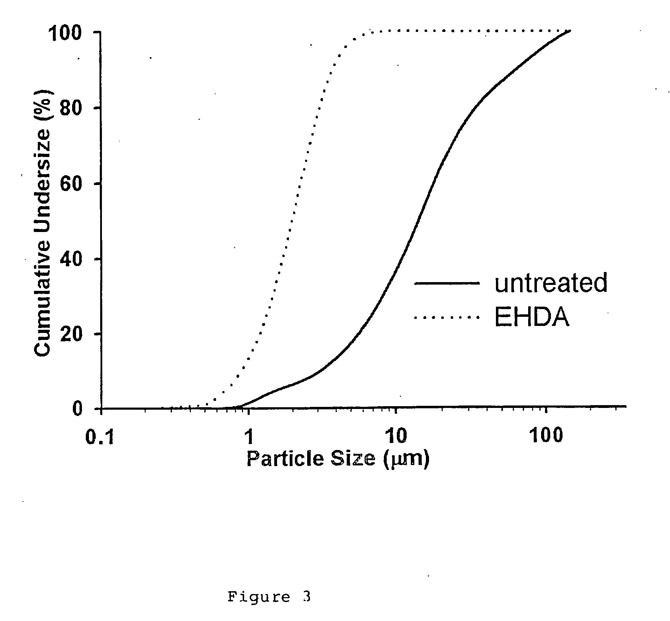

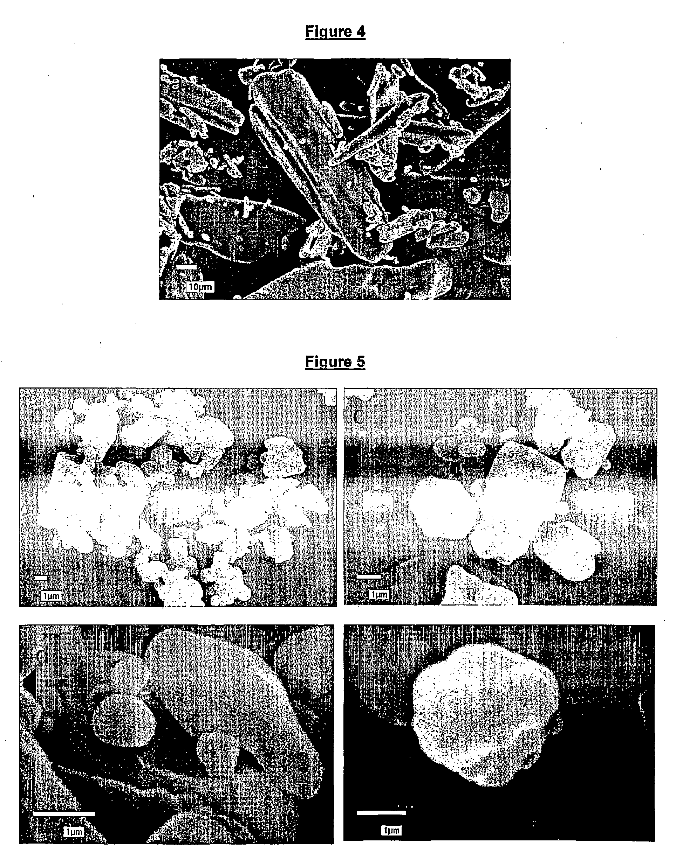

Figure 3a shows the cumulative size distribution for the original paracetamol particles used to make up the 7.5%w/w solution. The original paracetamol particles are clearly polydispersed. Figure 3b shows the cumulative size distribution for the particles produced by electrohydrodynamic spraying and sonocrystallisation. The particles produced by electrohydrodynamic spraying and sonocrystallisation have a narrow size distribution between $1\mu m$ and $5\mu m$. The crystallinity of the particles produced by electrohydrodynamic spraying and sonocrystallisation was verified by differential scanning calorimetry and X-ray powder diffraction. Figure 4 shows a scanning electron micrograph of the original paracetamol particles and Figure 5 those produced by electrohydrodynamic spraying and sonocrystallisation. The particles produced by the process of the invention are substantially spherical, and this contrast with the original particles can be clearly seen.

**Example 2 - Preparation of Crystalline Paracetamol Particles and Beclomethasone Dipropionate (BDP) particles**

A high pressure atomisation system, as described above, was used to produce paracetamol particles and beclomethasone dipropionate (BDP) particles.

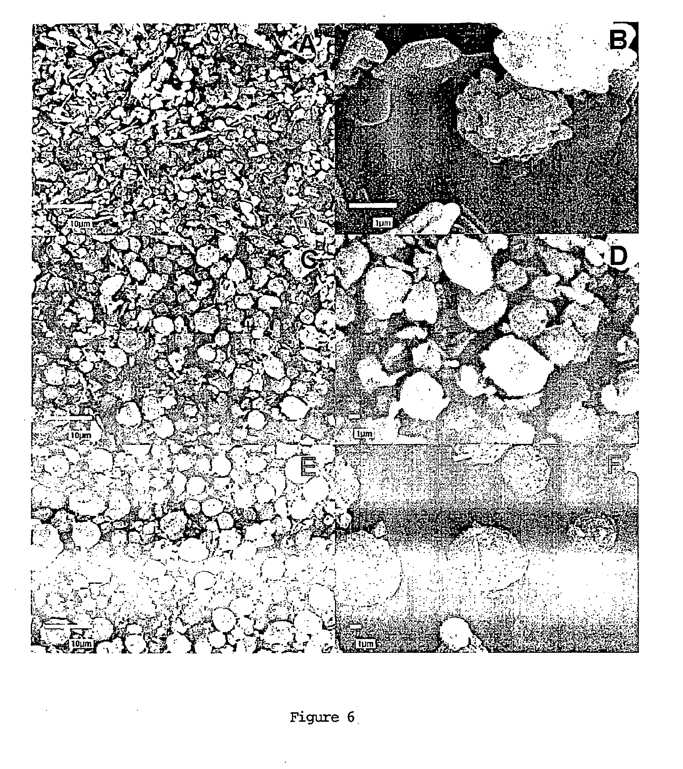

A solution of paracetamol in ethanol was sprayed through a 0.7mm diameter orifice with a supporting air flow rate of 600 l/h. The flow rate of the solvent was controlled by a syringe pump and was set at 16 ml/h. The aerosol droplets were collected in cyclohexane via a conical shaped crystallisation vessel. The distance between the atomiser orifice and the collection vessel was pre-set at well-defined separation distances. A typical separation distance was around 15cm. The whole system was hermetically sealed. A side arm attached to the flask provided a flow path of the air through the system. Nucleation of the droplets collected in the crystallisation vessel was induced via ultrasonic energy. The crystalline particles were collected by filtration and washed with a non-solvent via a 0.22pm and subsequently dried. Figure 6 shows scanning electron micrographs of paracetamol particles produced by the air pressure atomisation system with increasing drug concentration (1% w/w, 5% w/w and 10% w/w respectively).

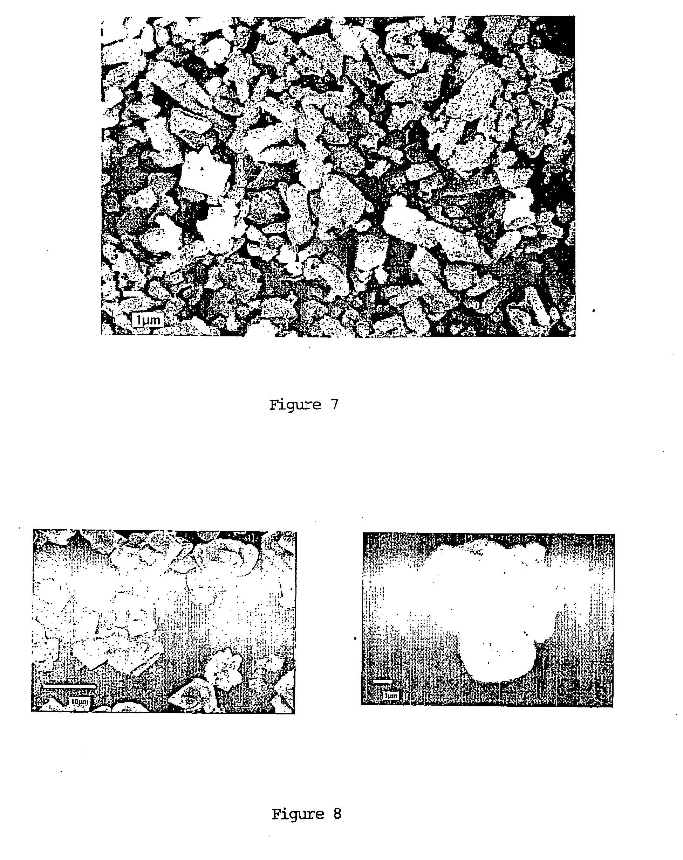

The use of the high pressure atomisation system was also utilised for the production of Beclomethasone dipropionate particles. Figure 7 shows a scanning electron micrograph of the particles produced from a 2% w/w BDP-ethanol solution. The experimental conditions of the high pressure atomisation system were the same as for paracetamol.

**Example 3 - Statistical Analysis Comparison of Micronized Particles with Particles Produced By the Process of the Invention**

For statistical analysis, the cumulative percentage is plotted on a probability scale (ordinate) versus logarithm of the particle size (abscissa). In most cases, the cumulative frequency distributions tend to follow a log-normal distribution. Differences in particle size distributions were characterised by parameters derived from comparing certain percentiles values from each distribution. The computed distribution parameters were median separation energy, skewness, and kurtosis.

The central tendency of each distribution is characterised by its median separation energy, the energy required to detach 50% of the adhered particles. The Yule coefficient of skewness measures the degree of deviation of a distribution from symmetry. The non-dimensional quantity can take any value between -1 and 1. Where skewness is zero, an even distribution of data around one standard deviation of the median is suggested. For a strongly non-zero measurement, an asymmetric, skewed distribution is suggested. Distributions with a positive skewness are skewed towards the high particle size tail, whereas distributions with negative skewness are skewed towards the lower particle size tail. Kurtosis denotes the shape of the distribution about the centre. The coefficient of kurtosis has a value between 0.99 an 1.11 for a normal type distribution (mesokurtic), while more negative values indicate an increase in the flatness of the frequency distribution (platykurtic), and more positive values show an increased peakness for the particle size distribution (leptokurtic). The various expressions used to calculate the statistical parameters are provided below.

Skewness

$$Sk = \frac{(e_{84.1\%} - 2e_{50\%} + e_{15.9\%})}{(e_{84.1\%} - e_{15.9\%})}$$

Kurtosis

$$ku = \sqrt{\frac{e_{15.9\%}/e_{84.1\%}}{e_{75\%}/e_{25\%}}}$$

Table 6: Analysis of micronised paracetamol and controlled atomisation and sonocrystallization of paracetamol

|  | Micronised | EHD Sprayed | High Pressure Atomisation |
|---|---|---|---|
| Median Diameter/$\mu$m | 2.74 | 2.31 | 3.05 |
| Skewness | 0.381 | 0.250 |  |
| Kurtosis | 0.260 | 0.425 |  |

Table 7: Analysis of micronised and controlled atomisation and crystallization of beclomethasone dipropionate

|  | Micronised | High pressure atomisation |
|---|---|---|
| Median Diameter/$\mu$m | 3.33 | 1.20 |
| Skewness | 0.326 | 0.266 |
| Kurtosis | 0.321 | 0.401 |

The results clearly show that the particles produced by the process of the present invention have a lower skewness (i.e. a more symmetrical distribution) and a higher kurtosis (i.e. a less dispersed distribution) than particles produced by prior art processes. These parameters lead to improved utility in pharmaceutical compositions.

**Example 4: Preparation of crystalline budesonide particles**

A high pressure atomisation system, as described previously, was used to produce budesonide particles within a well-defined particle size range. Figure 8 shows a scanning electron micrograph of the particles produced upon spraying 8% w/w budesonide solution in dichloromethane through a 0.7mm diameter orifice with a supporting air flow rate of 600 l/h. The flow rate of the solvent was set at 40ml/hour. The aerosol droplets were collected in hexane at a separation distance of 20cm.

**Claims**

1. A process for the production of particles comprising the steps of

    i) formation of a solution of a desired substance in a suitable solvent;
    ii) generation of an aerosol from the solution of said substance;
    iii) collection of the aerosol droplets in a vessel containing a non-solvent of said substance; and
    iv) application of ultrasound to the droplets dispersed in the non-solvent to effect crystallisation of said substance.

2. A process according to claim 1 wherein the solvent evaporates from the aerosol droplets between generation of the aerosol and collection of the aerosol droplets in the non-solvent.

3. A process as claimed in claim 2 wherein at least 80% by mass of each collected droplet is molecules of the desired substance.

4. A process as claimed in any preceding claim wherein the concentration of the pharmaceutically acceptable substance in the solution formed in step i) of the process is from 50 mg/ml to 200 mg/ml.

5. A process as claimed in claim 4 wherein the solution is a saturated solution of the desired substance.

6. A process as claimed in any preceding claim wherein the method of aerosol generation is a high air pressure atomiser or an electrohydrodynamic spray atomiser.

7. A process as claimed in any preceding claim wherein the droplets produced by the aerosol generator have an initial diameter between 1 $\mu$m and 50 $\mu$m.

8. A process as claimed in any one of claims 1 to 6 for the production of nanometre size particles wherein the droplets produced by the aerosol generator have an initial diameter between 10 nm and 1 μm.

9. A process as claimed in any preceding claim wherein the non-solvent in which the aerosol droplets are collected contains an emulsifier.

10. A process as claimed in any preceding claim wherein the desired substance is a pharmaceutically acceptable substance or an agrochemically active substance.

11. A process as claimed in claim 10 wherein the desired substance is a drug.

12. A process as claimed in claim 11 wherein the drug is suitable for use in an inhalation formulation.

13. A process as claimed in claim 12 wherein the drug is salbutamol.

14. Crystalline particles, suitable for use in a pharmaceutical formulation, **characterised in that** the particles are spherical and have nanometre scale surface corrugations, wherein the particles are drug particles suitable for use in an inhalation formulation, the particles being obtainable by the process of any one of claims 1-13.

15. A pharmaceutical composition comprising particles as claimed in claim 14.

16. Use of crystalline particles as claimed in claim 14 for the manufacture of a medicament.


**Patentansprüche**

1. Verfahren zur Herstellung von Partikeln, umfassend die folgenden Schritte:

i) Bilden einer Lösung einer gewünschten Substanz in einem geeigneten Lösungsmittel;
ii) Erzeugen eines Aerosols aus der Lösung dieser Substanz;
iii) Auffangen der Aerosoltropfen in einem Gefäß, das ein Nicht-Lösungsmittel dieser Substanz enthält; und
iv) Anwenden von Ultraschall an den in dem Nicht-Lösungsmittel dispergierten Tropfen, um die Kristallisierung dieser Substanz zu bewirken.

2. Verfahren nach Anspruch 1, wobei das Lösungsmittel zwischen der Erzeugung des Aerosols und dem Auffangen der Aerosoltropfen in dem Nicht-Lösungsmittel aus den Aerosoltropfen verdampft.

3. Verfahren nach Anspruch 2, wobei mindestens 80 Massen-% von jedem aufgefangenen Tropfen Moleküle der gewünschten Substanz sind.

4. Verfahren nach einem der vorhergehenden Ansprüche, wobei die Konzentration der pharmazeutisch zulässigen Substanz in der in Schritt i) des Verfahrens gebildeten Lösung von 50 mg/ml bis 200 mg/ml beträgt.

5. Verfahren nach Anspruch 4, wobei die Lösung eine gesättigte Lösung der gewünschten Substanz ist.

6. Verfahren nach einem der vorhergehenden Ansprüche, wobei das Verfahren der Aerosolerzeugung ein Hochdruckzerstäuber oder ein elektrohydrodynamischer Sprühzerstäuber ist.

7. Verfahren nach einem der vorhergehenden Ansprüche, wobei die durch den Aerosolerzeuger hergestellten Tropfen einen Anfangsdurchmesser zwischen 1 μm und 50 μm aufweisen.

8. Verfahren nach einem der Ansprüche 1 bis 6 zur Herstellung von nanometergroßen Partikeln, wobei die durch den Aerosolerzeuger hergestellten Tropfen einen Anfangsdurchmesser zwischen 10 nm und 1 μm aufweisen.

9. Verfahren nach einem der vorhergehenden Ansprüche, wobei das Nicht-Lösungsmittel, in dem die Aerosoltropfen aufgefangen werden, einen Emulgator enthält.

10. Verfahren nach einem der vorhergehenden Ansprüche, wobei die gewünschte Substanz eine pharmazeutisch zulässige Substanz oder eine agrochemisch aktive Substanz ist.

**11.** Verfahren nach Anspruch 10, wobei die gewünschte Substanz ein Wirkstoff ist.

**12.** Verfahren nach Anspruch 11, wobei der Wirkstoff für eine Verwendung in einer Inhalationsformulierung geeignet ist.

**13.** Verfahren nach Anspruch 12, wobei der Wirkstoff Salbutamol ist.

**14.** Kristalline Partikel, die für die Verwendung in einer pharmazeutischen Formulierung geeignet sind, **dadurch gekennzeichnet, dass** die Partikel kugelförmig sind und nanometergroße Oberflächenwellungen aufweisen, wobei die Partikel Wirkstoffpartikel sind, die für eine Verwendung in einer Inhalationsformulierung geeignet sind, wobei die Partikel durch das Verfahren nach einem der Ansprüche 1-13 erhalten werden können.

**15.** Pharmazeutische Zusammensetzung, umfassend Partikel nach Anspruch 14.

**16.** Verwendung von kristallinen Partikeln nach Anspruch 14 zur Herstellung eines Medikaments.


**Revendications**

**1.** Procédé pour la production de particules comprenant les étapes de :

i) formation d'une solution d'une substance souhaitée dans un solvant approprié ;
ii) génération d'un aérosol à partir de la solution de ladite substance ;
iii) collecte des gouttelettes d'aérosol dans un récipient contenant un non-solvant de ladite substance ; et
iv) application d'ultrasons aux gouttelettes dispersées dans le non-solvant pour effectuer une cristallisation de ladite substance.

**2.** Procédé selon la revendication 1, dans lequel le solvant s'évapore à partir des gouttelettes d'aérosol entre la génération de l'aérosol et la collecte des gouttelettes d'aérosol dans le non-solvant.

**3.** Procédé selon la revendication 2, dans lequel au moins 80 % en masse de chaque gouttelette collectée sont des molécules de la substance souhaitée.

**4.** Procédé selon l'une quelconque des revendications précédentes, dans lequel la concentration de la substance pharmaceutiquement acceptable dans la solution formée dans l'étape i) du procédé est de 50 mg/ml à 200 mg/ml.

**5.** Procédé selon la revendication 4, dans lequel la solution est une solution saturée de la substance souhaitée.

**6.** Procédé selon l'une quelconque des revendications précédentes, dans lequel le procédé de génération d'aérosol est un atomiseur à pression d'air élevée ou un atomiseur par pulvérisation électrohydrodynamique.

**7.** Procédé selon l'une quelconque des revendications précédentes, dans lequel les gouttelettes produites par le générateur d'aérosol ont un diamètre initial entre 1 $\mu$m et 50 $\mu$m.

**8.** Procédé selon l'une quelconque des revendications 1 à 6 pour la production de particules de dimension nanométrique, dans lequel les gouttelettes produites par le générateur d'aérosol ont un diamètre initial entre 10 nm et 1 $\mu$m.

**9.** Procédé selon l'une quelconque des revendications précédentes, dans lequel le non-solvant dans lequel les gouttelettes d'aérosol sont collectées contient un émulsifiant.

**10.** Procédé selon l'une quelconque des revendications précédentes, dans lequel la substance souhaitée est une substance pharmaceutiquement acceptable ou une substance active en agrochimie.

**11.** Procédé selon la revendication 10, dans lequel la substance souhaitée est un médicament.

**12.** Procédé selon la revendication 11, dans lequel le médicament est approprié en vue d'une utilisation dans une formulation pour inhalation.

**13.** Procédé selon la revendication 12, dans lequel le médicament est le salbutamol.

**14.** Particules cristallines, appropriées en vue d'une utilisation dans une formulation pharmaceutique, **caractérisées en ce que** les particules sont sphériques et ont des ondulations de surface d'échelle nanométrique, dans lesquelles les particules sont des particules de médicament appropriées en vue d'une utilisation dans une formulation pour inhalation, les particules pouvant être obtenues par le procédé de l'une quelconque des revendications 1-13.

**15.** Composition pharmaceutique comprenant des particules telles que revendiquées dans la revendication 14.

**16.** Utilisation de particules cristallines telles que revendiquées dans la revendication 14 pour la fabrication d'un médicament.

Figure 1

Figure 2

Figure 3

**Figure 4**

**Figure 5**

Figure 6

Figure 7

Figure 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 02089942 A